(19) Europäisches Patentamt
European Patent Office
Office européen des brevets

(11) EP 4 171 558 B1

(12) **EUROPEAN PATENT SPECIFICATION**

(45) Date of publication and mention of the grant of the patent:
**24.09.2025 Bulletin 2025/39**

(21) Application number: **21830102.6**

(22) Date of filing: **25.06.2021**

(51) International Patent Classification (IPC):
***A61K 31/44*** *(2006.01)* ***A61K 31/4409*** *(2006.01)*
***C08F 220/54*** *(2006.01)* ***C08F 220/56*** *(2006.01)*
***C08F 22/38*** *(2006.01)* ***C08F 120/60*** *(2006.01)*
***C09D 133/24*** *(2006.01)*

(52) Cooperative Patent Classification (CPC):
**C09D 133/14; C08F 22/38; C08F 120/60; C09D 133/24**

(86) International application number:
**PCT/US2021/039026**

(87) International publication number:
**WO 2021/263074 (30.12.2021 Gazette 2021/52)**

(54) **ANTIMICROBIAL COMPOUNDS AND COMPOSITIONS**

ANTIMIKROBIELLE VERBINDUNGEN UND ZUSAMMENSETZUNGEN

COMPOSÉS ANTIMICROBIENS ET COMPOSITIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.06.2020 US 202063044139 P**

(43) Date of publication of application:
**03.05.2023 Bulletin 2023/18**

(73) Proprietor: **Prevenial, LLC**
**Orlando, FL 32801 (US)**

(72) Inventor: **GREENHOE, Brian, M.**
**Knoxville, TN 37919 (US)**

(74) Representative: **Carpmaels & Ransford LLP**
**One Southampton Row**
**London WC1B 5HA (GB)**

(56) References cited:
**EP-A1- 0 980 682**
**WO-A1-2021/094960**
**JP-A- H08 157 318**
**US-A- 6 153 660**
**US-A1- 2012 022 069**

- **MORIMOTO NOBUYUKI, OISHI YOSHIFUMI, YAMAMOTO MASAYA: "The Design of Sulfobetaine Polymers with Thermoresponsiveness under Physiological Salt Conditions", MACROMOLECULAR CHEMISTRY AND PHYSICS, vol. 221, no. 5, 1 March 2020 (2020-03-01), DE , pages 1 - 7, XP055896777, ISSN: 1022-1352, DOI: 10.1002/macp.201900429**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 171 558 B1

**Description**

TECHNICAL FIELD

The present disclosure pertains to compounds and compositions having antimicrobial properties.

BACKGROUND

Microbial contamination of materials that are intended for use in such fields as medical treatment, manufacture and packaging of medical equipment, consumer products, food preparation or packaging, and the like, represents a longstanding concern. Sterilization techniques may be temporarily effective, and protocols for the manufacture and use of materials that are intended to remain sterile have proven to be imperfect.

Alternative approaches for reducing microbial load on such materials include antimicrobial coatings, or compositions that are incorporated into the materials themselves in order to confer antimicrobial properties. A need persists for further approaches for conferring antimicrobial properties on materials for use in relevant fields.

SUMMARY

Disclosed herein are compounds of Formula (I):

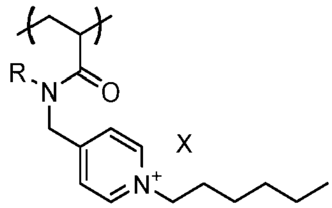

(I)

wherein

R is H, -OH, $-CH_2CH_3$, $-(CH_2)_2CH_3$, $-(CH_2)_3CH_3$, or $-(CH_2)_2OCH_3$; and,
X is a negative counterion that is bromide, chloride, fluoride, or a phosphate, where the compound is a homopolymer.

Also disclosed are compositions comprising a compound of Formula (I) and a base material. The present disclosure also provides articles comprising such compositions.

The present disclosure also pertains to methods for preparing a compound according to Formula (I). Also provided are methods comprising combining a base polymeric material with a compound of a Formula (I).

DETAILED DESCRIPTION OF ILLUSTRATIVE EMBODIMENTS

The present inventions may be understood more readily by reference to the following detailed description taken in connection with the accompanying examples, which form a part of this disclosure. It is to be understood that these inventions are not limited to the specific formulations, methods, articles, or parameters described and/or shown herein, and that the terminology used herein is for the purpose of describing particular embodiments by way of example only and is not intended to be limiting of the claimed inventions.

As employed above and throughout the disclosure, the following terms and abbreviations, unless otherwise indicated, shall be understood to have the following meanings.

In the present disclosure the singular forms "a," "an," and "the" include the plural reference, and reference to a particular numerical value includes at least that particular value, unless the context clearly indicates otherwise. Thus, for example, a reference to "a material" is a reference to one or more of such materials and equivalents thereof known to those skilled in the art, and so forth. Furthermore, when indicating that a certain element "may be" X, Y, or Z, it is not intended by such usage to exclude in all instances other choices for the element.

When values are expressed as approximations, by use of the antecedent "about," it will be understood that the particular value forms another embodiment. As used herein, "about X" (where X is a numerical value) preferably refers to $\pm$10% of the recited value, inclusive. For example, the phrase "about 8" can refer to a value of 7.2 to 8.8, inclusive. This value may include "exactly 8". Where present, all ranges are inclusive and combinable. For example, when a range of "1 to 5" is recited, the recited range should be construed as optionally including ranges "1 to 4", "1 to 3", "1-2", "1-2 & 4-5", "1-3 & 5", and the like. In addition, when a list of alternatives is positively provided, such a listing can also include embodiments

where any of the alternatives may be excluded. For example, when a range of "1 to 5" is described, such a description can support situations whereby any of 1, 2, 3, 4, or 5 are excluded; thus, a recitation of "1 to 5" may support "1 and 3-5, but not 2", or simply "wherein 2 is not included."

Microbial (e.g., bacterial) contamination can have severe consequences, whether in connection with medical equipment or materials that come in contact with food or pharmaceutical ingredients. When used to manufacture items like disposable gloves or medical tubing, materials such as polyvinyl chloride (PVC), nylon, or polycarbonate can be susceptible to microbial contamination. Previous efforts to confer antimicrobial properties materials that are commonly used in medical and other contexts in which microbial contamination presents problems have focused on both surface coatings and additives to the materials themselves. The present inventors have discovered novel compounds that can be easily combined with commonly used materials in order to confer antimicrobial properties. The compounds are versatile, easy to manufacture, and highly efficacious even when used in low concentrations.

Provided herein are compounds according to Formula (I):

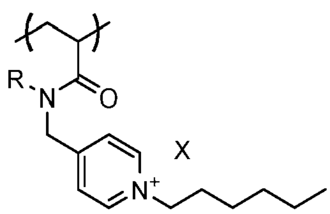

(I)

wherein

R is H, -OH, $-CH_2CH_3$, $-(CH_2)_2CH_3$, $-(CH_2)_3CH_3$, or $-(CH_2)_2OCH_3$; and,

X is a negative counterion that is bromide, chloride, fluoride, or a phosphate, wherein the compound is a homopolymer.

In certain embodiments, X is bromide. With respect to some of these embodiments, R is $-CH_2CH_3$, $-(CH_2)_2CH_3$, or $-(CH_2)_3CH_3$. For example, in one embodiment, X is bromide, and R is $-CH_2CH_3$.

In final form, the compounds represent homopolymers that may have a molecular weight of about 10 kDa to about 200 kDa. For example, the homopolymeric compounds may have a molecular weight of about 10, 20, 30, 40, 50, 60, 70, 80, 90, 100, 110, 120, 130, 140, 150, 160, 170, 180, 190, or 200 kDa.

Also provided are mixtures comprising at least two different compounds according to Formula (I). For example, a mixture according to the present disclosure may include a compound according to Formula (I) in which X is bromide and R is $-CH_2CH_3$, and a compound according to Formula (I) in which X is bromide and R is -OH. In one embodiment, the mixture includes a compound according to Formula (I) in which X is bromide and R is $-CH_2CH_3$, and at least one additional compound of Formula (I).

In some embodiments, the present mixtures may represent a single compound of Formula (I) having a first molecular weight, in combination with another compound having the same substituents as the first compound (*i.e.,* wherein variable groups R and X are the same as the first compound) but having a molecular weight that is different from that of the first compound. For example, a mixture according to the present disclosure may include a first compound in which X is bromide and R is ethyl and having a molecular weight of about 50 kDa, and a second compound in which X is bromide and R is ethyl and having a molecular weight of about 150 kDa.

As used throughout the present disclosure, "antimicrobial" refers to the ability to reduce microbial load by a measurable degree, or to prevent or diminish microbial contamination that would otherwise occur. For example, an antimicrobial compound according to the present disclosure can reduce microbial load with respect to a base material with which it is mixed, or can prevent persistent microbial contamination with respect to the base material.

When combined with a base material, compounds of the disclosure are effective in reducing the number of pathogens in or on the base material as compared to a base material that has not been combined with a present compound. The antimicrobial efficacy of the present compounds may be expressed in terms of Minimal Inhibitory Concentration (MIC), Minimal Bactericidal Concentration (MBC), or both. The MIC of the present compounds may be, for example, no more than about 0.5%, 0.45%, 0.4%, 0.35%, 0.3%, 0.25%, 0.2%, 0.19%, 0.18%, 0.17%, 0.16%, or 0.15% as measured in accordance with ISO 20776-1:2019. The MBC of the present compounds may be, for example, no more than about 0.5%, 0.45%, 0.4%, 0.35%, 0.3%, 0.25%, 0.2%, 0.19%, 0.18%, 0.17%, 0.16%, or 0.15%, 0.14%, 0.13%, 0.12%, 0.11%, 0.1%, 0.05%, 0.01%, 0.005%, or 0.0005%, as measured in accordance with ISO 20776-1:2019.

Microbes against which the present compounds are effective to cause reduction in numbers may be, for example, any unicellular organism, such as gram-negative bacteria, gram-positive bacteria, protozoa, viruses, bacteriophages, and archaea. The present compounds can have an antimicrobial effect with respect to any such microbe. Examples of bacteria against which the present compounds are effective to cause reduction in numbers include gram positive bacteria and gram

negative bacteria, for example, Salmonella enterica, Listeria monocytogenes, Escherichia coli, Clostridium botulinum, Clostridium difficile, Campylobacter, Bacillus cereus, Vibrio parahaemolyticus, Vibrio cholerae, Vibrio vulnificus, Staphylococcus aureus, Yersinia enterocolitica, Shigella, Moraxella spp., Helicobacter, Stenotrophomonas, Bdellovibrio, Legionella spp. (*e.g.,* pneumophila), Neisseria gonorrhoeae, Neisseria meningitidis, Haemophilus influenzae, Acinetobacter baumannii, Klebsiella pneumoniae, Pseudomonas aeruginosa, Proteus mirabilis, Enterobacter cloacae, Serratia marcescens, Helicobacter pylori, Salmonella enteritidis, Salmonella typhi, and combinations thereof. Examples of Salmonella enterica serovars that can be reduced using the compounds of the disclosure include, for example, Salmonella enteriditis, Salmonella typhimurium, Salmonella poona, Salmonella heidelberg, and Salmonella anatum. Exemplary viruses against which the present compounds are effective to cause reduction in numbers include coronaviruses, rhinoviruses, and influenza viruses.

The present disclosure also provides compositions that include a compound according to Formula (I) and a base material. The compound of Formula (I) may be any compound or mixture of such compounds as defined *supra.* The base material may be any material that is used to form, at least in part, an article for use in medical diagnosis or treatment, including tubing, delivery devices, or protective clothing used by medical practitioners, a packaging article, such as for use in packaging a pharmaceutical or food product, or any other base material concerning which it is desirable to avoid microbial contamination.

For example, the base material may be polyvinyl chloride, chlorinated polyvinyl chloride, polycarbonate, nylon 6-6, nylon 6, nylon 10, polyethylene terephthalate, polyethylene terephthalate glycol, polybutylene terephthalate, polyether block amide, acrylate, acrylonitrile butadiene styrene, polystyrene, polylactic acid, polyhydroxyalkanoate, polyoxymethylene, low density polyethylene, high density polyethylene, polypropylene, ethylene-vinyl acetate, acrylonitrile styrene acrylate, epoxies, silicones, latex, or any other commodity bulk material, e.g., commodity bulk plastic, or any combination or mixture thereof. For example, the base material may be nitrile rubber, which is a copolymer of acrylonitrile and butadiene.

The compound of Formula (I) may be present in the composition in an amount of about 0.05 to 15 wt%. In some embodiments, the compound of Formula (I) is present in the composition in an amount of about 0.05-13, 0.1-15, 0.1-10, 0.2-13, 0.3-15, 0.3-13, 0.3-10, 0.4-15, 0.4-13, 0.4-10, 0.5-10, 1-10, 1-9, 1-8, 2-7, 3-7, 3-6, or 3-5 wt%. For example, the compound of Formula (I) may be present in the composition in an amount of about 0.05, 0.07, 0.09. 0.1, 0.2, 0.3, 0.4, 0.5, 0.75, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or 15 wt%.

The present compositions may optionally include one or more additional components that, for example, improve the processability or end-use performance of the compositions, such as one or more fillers, plasticizers, ultraviolet absorbers, antioxidants, stabilizers, thickeners, colorants, rheological agents, or the like.

Fillers can be selected to impart additional strength or provide additional characteristics that can be based on the final selected characteristics of the composition. In some aspects, the filler(s) can comprise inorganic materials which can include clay, titanium oxide, asbestos fibers, silicates and silica powders, boron powders, calcium carbonates, talc, kaolin, sulfides, barium compounds, metals and metal oxides, wollastonite, glass spheres, glass fibers, flaked fillers, fibrous fillers, natural fillers and reinforcements, and reinforcing organic fibrous fillers. In certain aspects, the composite may comprise a glass fiber filler. In yet further aspects, the composite may be free or substantially free of a glass filler.

Appropriate fillers or reinforcing agents can include, for example, mica, clay, feldspar, quartz, quartzite, perlite, tripoli, diatomaceous earth, aluminum silicate (mullite), synthetic calcium silicate, fused silica, fumed silica, sand, boron-nitride powder, boron-silicate powder, calcium sulfate, calcium carbonates (such as chalk, limestone, marble, and synthetic precipitated calcium carbonates) talc (including fibrous, modular, needle shaped, and lamellar talc), wollastonite, hollow or solid glass spheres, silicate spheres, cenospheres, aluminosilicate or (armospheres), kaolin, whiskers of silicon carbide, alumina, boron carbide, iron, nickel, or copper, continuous and chopped carbon fibers or glass fibers, molybdenum sulfide, zinc sulfide, barium titanate, barium ferrite, barium sulfate, heavy spar, titanium dioxide, aluminum oxide, magnesium oxide, particulate or fibrous aluminum, bronze, zinc, copper, or nickel, glass flakes, flaked silicon carbide, flaked aluminum diboride, flaked aluminum, steel flakes, natural fillers such as wood flour, fibrous cellulose, cotton, sisal, jute, starch , lignin, ground nut shells, or rice grain husks, reinforcing organic fibrous fillers such as poly(ether ketone), polyimide, polybenzoxazole, poly(phenylene sulfide), polyesters, polyethylene, aromatic polyamides, aromatic polyimides, polyetherimides, polytetrafluoroethylene, and poly(vinyl alcohol), as well combinations comprising at least one of the foregoing fillers or reinforcing agents. The fillers and reinforcing agents can be coated or surface treated, with silanes for example, to improve adhesion and dispersion with the polymer matrix. Fillers generally can be used in amounts of 1 to 200 parts by weight, based on 100 parts by weight of based on 100 parts by weight of the total composition.

The composition can also comprise plasticizers. For example, plasticizers can include phthalic acid esters such as dioctyl-4,5-epoxy-hexahydrophthalate, tris-(octoxycarbonylethyl) isocyanurate, tristearin, epoxidized soybean oil or the like, or combinations including at least one of the foregoing plasticizers. Plasticizers are generally used in amounts of from about 0.5 to about 3.0 parts by weight, based on 100 parts by weight of the total composition, excluding any filler.

Ultraviolet (UV) absorbers can also be present in the disclosed compositions. Exemplary ultraviolet absorbers can include for example, hydroxybenzophenones; hydroxybenzotriazoles; hydroxybenzotriazines; cyanoacrylates;

oxanilides; benzoxazinones; 2- (2H-benzotriazol-2-yl)-4-(1,1,3,3-tetramethylbutyl)-phenol (CYASORB™ 5411); 2-hydroxy-4-n-octyloxybenzophenone (CYASORB™ 531); 2-[4,6-bis(2,4-dimethylphenyl)-1,3,5-triazin-2-yl]- 5-(octyloxy)-phenol (CYASORB™ 1164); 2,2'-(1,4- phenylene)bis(4H-3,1-benzoxazin-4-one) (CYASORB™ UV- 3638); 1,3-bis[(2-cyano-3,3-diphenylacryloyl)oxy]-2,2-bis[[(2-cyano-3, 3-diphenylacryloyl)oxy]methyl]propane (UVINUL™ 3030); 2,2'-(1,4-phenylene) bis(4H-3,1-benzoxazin-4-one); 1,3-bis[(2-cyano-3,3-diphenylacryloyl)oxy] -2,2-bis[[(2-cyano-3,3-diphenylacryloyl)oxy]methyl]propane; nano-size inorganic materials such as titanium oxide, cerium oxide, and zinc oxide, all with particle size less than 100 nanometers; or the like, or combinations including at least one of the foregoing UV absorbers. UV absorbers are generally used in amounts of from 0.01 to 3.0 parts by weight, based on 100 parts by weight of the total composition, excluding any filler.

In further aspects, one or more light stabilizers can be present in the present compositions. Exemplary light stabilizers can include, for example, benzotriazoles such as 2-(2-hydroxy-5-methylphenyl)benzotriazole, 2-(2-hydroxy-5-tert-octylphenyl)-benzotriazole and 2-hydroxy-4-n-octoxy benzophenone or the like or combinations including at least one of the foregoing light stabilizers. Light stabilizers can generally be used in amounts of from about 0.1 to about 1.0 parts by weight, based on 100 parts by weight of the total composition, excluding any filler.

The present compositions can comprise a heat stabilizer. As an example, heat stabilizers can include, for example, organo phosphites such as triphenyl phosphite, tris-(2,6-dimethylphenyl)phosphite, tris-(mixed mono-and di-nonylphenyl)phosphite or the like; phosphonates such as dimethylbenzene phosphonate or the like, phosphates such as trimethyl phosphate, or the like, or combinations including at least one of the foregoing heat stabilizers. Heat stabilizers can generally be used in amounts of from 0.01 to 0.5 parts by weight based on 100 parts by weight of the total composition, excluding any filler.

The composition can comprise one or more antioxidants. The antioxidants can include either a primary or a secondary antioxidant. For example, antioxidants can include organophosphites such as tris(nonyl phenyl)phosphite, tris(2,4-di-t-butylphenyl)phosphite, bis(2,4-di-t-butylphenyl)pentaerythritol diphosphite, distearyl pentaerythritol diphosphite or the like; alkylated monophenols or polyphenols; alkylated reaction products of polyphenols with dienes, such as tetrakis[methylene(3,5-di-tert-butyl-4-hydroxyhydrocinnamate)] methane, or the like; butylated reaction products of para-cresol or dicyclopentadiene; alkylated hydroquinones; hydroxylated thiodiphenyl ethers; alkylidene-bisphenols; benzyl compounds; esters of beta-(3,5-di-tert-butyl-4-hydroxyphenyl)-propionic acid with monohydric or polyhydric alcohols; esters of beta-(5-tert-butyl-4-hydroxy-3-methylphenyl)-propionic acid with monohydric or polyhydric alcohols; esters of thioalkyl or thioaryl compounds such as distearylthiopropionate, dilaurylthiopropionate, ditridecylthiodipropionate, octadecyl-3-(3,5-di-tert-butyl-4-hydroxyphenyl)propionate, pentaerythrityl-tetrakis[3-(3,5-di-tert-butyl-4-hydroxyphenyl)propionate or the like; amides of beta-(3,5-di-tert-butyl-4-hydroxyphenyl)-propionic acid or the like, or combinations including at least one of the foregoing antioxidants. Antioxidants can generally be used in amounts of from 0.01 to 0.5 parts by weight, based on 100 parts by weight of the total composition, excluding any filler.

The present compositions may comprise a pigment or colorant. Suitable colorants may include, but are not limited to, Solvent Green 3, Solvent Green 28, Solvent Green 38, Pigment Green 50, Pigment Green 36, Solvent Red 52, Solvent Red 101, Solvent Red 111, Solvent Red 135, Solvent Red 169, Solvent Red 179, Solvent Red 207, Pigment Red 101, Disperse Red 22, Vat Red 41, Solvent Orange 60, Solvent Orange 63, Disperse Orange 47, Solvent Violet 13, Solvent Violet 14, Solvent Violet 36, Solvent Violet 50, Disperse violet 26/31, Pigment Blue 29, Pigment Blue 60, Copper Phthalocyanine Pigment Blue 15.4, Disperse Blue 73, Solvent Blue 97, Solvent Blue 101, Solvent Blue 104, Solvent Blue 122, Solvent Blue 138, Pigment Yellow 53, Pigment Yellow 138, Pigment Yellow 139, Disperse Yellow 201, Solvent Yellow 33, Solvent Yellow 114, Solvent Yellow 93, Solvent Yellow 98, Solvent Yellow 163, Solvent Yellow 160:1, Solvent Yellow 188, Pigment Brown 24, Amino Ketone Black, chrome oxides, carbon black, channel black, and pigment black 6and the like, as well as combinations including one or more of the foregoing. Any effective amount of the colorant may be included in the molded article. In some aspects the colorant is present in the molded article in an amount of from about 0.00001 to about 0.01 wt. % of the composition, or in certain aspects in an amount of from about 0.00002 to about 0.0010 wt. % of the composition, or even in an amount of from about 0.00002 to about 0.0005 wt. % of the composition.

The compositions may comprise one or more pigments, such as a white pigment. A white pigment can impart opacity or a bright opaque appearance. Examples of white pigments may include titanium dioxide, zinc sulfide (ZnS), tin oxide, aluminum oxide ($AlO_3$), zinc oxide (ZnO), calcium sulfate, barium sulfate ($BaSO_4$), calcium carbonate (e.g., chalk), magnesium carbonate, antimony oxide ($Sb_2O_3$), white lead (a basic lead carbonate, $2PbCO_3 \cdot Pb(OH)_2$), lithopone (a combination of barium sulfate and zinc sulfide), sodium silicate, aluminum silicate, silicon dioxide ($SiO_2$, i.e., silica), mica, clay, talc, metal doped versions of the foregoing materials, and combinations including at least one of the foregoing materials. The pigment may be present in an amount of from about 0.1 wt. % to about 50 wt. %. As an example, the composition may include titanium dioxide in an amount of between 0.1 wt. % and 50 wt. %. In a further example, the composition may include titanium dioxide in an amount between 0.1 wt. % and 20 wt. %.

Also provided herein are articles that comprise a composition according to any of the disclosed embodiments. An article according to the present disclosure may be a bulk commodity material that is used, at least in part, to manufacture a final product, or the article may itself be a component of a product, or a finished product. For example, the article may be a

textile, or a sheet, ingot, pellet, or roll of material. In one embodiment, the article is a vinyl product, such as an upholstery vinyl. In another embodiment, the article is a natural product, such tanned or rawhide leather. If the article represents a finished product or a component of a finished product, the article may be, for example, a product that is used in medical treatment or otherwise by medical practitioners (e.g., doctors, dentists, nurses, hygienists, orderlies, assistants), such as a disposable glove, a protective mask, a medical gown, a shoe cover, a medical cap, medical tubing, a delivery device, a storage container, an implantable device, an implantable medical lead, a sponge, or a component thereof. The article may alternatively be a paint, sealant, coating, or wax, or a component thereof. The presence of the inventive compounds in the article confers antimicrobial characteristics, such that the article is less susceptible to microbial contamination and can be used more safely in critical contexts, such as medical treatment and food processing, storage, or service.

The present disclosure also provides methods for preparing a compound of Formula (I):

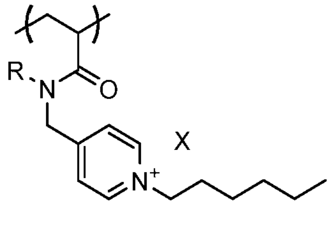

(I)

wherein

R is H, -OH, $-CH_2CH_3$, $-(CH_2)_2CH_3$, $-(CH_2)_3CH_3$, or $-(CH_2)_2OCH_3$; and,
X is a negative counterion that is bromide, chloride, fluoride, or a phosphate,
and, wherein the method is according to Scheme 1:

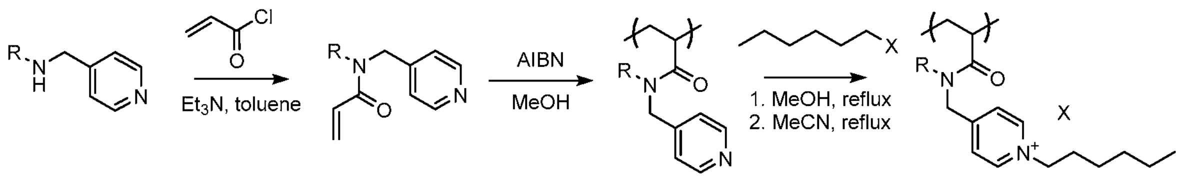


**Scheme 1**

The instant methods may be used to produce any compound according to Formula (I) as described *supra.* For example, the compound that the present methods produce may be such that X is bromide. With respect to some of these embodiments, R is - $CH_2CH_3$, $-(CH_2)_2CH_3$, or $-(CH_2)_3CH_3$. For example, in one embodiment, X is bromide, and R is $-CH_2CH_3$.

Additional aspects concerning the present methods are provided in connection with the examples that are provided below.

Also provided herein are methods comprising combining a base material with a compound of a Formula (I)

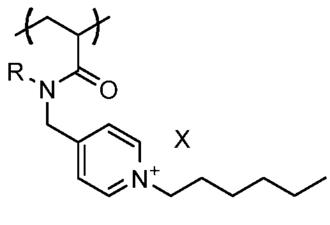

(I)

wherein R is H, -OH, $-CH_2CH_3$, $-(CH_2)_2CH_3$, $-(CH_2)_3CH_3$, or $-(CH_2)_2OCH_3$; and, X is a negative counterion that is bromide, chloride, fluoride, or a phosphate. The instant methods may involve the use of any compound according to Formula (I) as described *supra.* For example, the compound for use in the present method may be one in which X is bromide. With respect to some of these embodiments, R is $-CH_2CH_3$, $-(CH_2)_2CH_3$, or $-(CH_2)_3CH_3$. For example, in one embodiment, X is bromide, and R is $-CH_2CH_3$.

The base material may be any material that is described above in connection with the presently disclosed compositions. Thus, the base material may be any material used to form, at least in part, an article for use in medical diagnosis or treatment, including tubing, delivery devices, or protective clothing used by medical practitioners, a packaging article, such as for use in packaging a pharmaceutical or food product, or any other base material concerning which it is

desirable to avoid microbial contamination.

For example, the base material may be polyvinyl chloride, chlorinated polyvinyl chloride, polycarbonate, nylon 6-6, polyethylene terephthalate, polyethylene terephthalate glycol, polybutylene terephthalate, polyether block amide, acrylate, acrylonitrile butadiene styrene, polystyrene, polylactic acid, polyhydroxyalkanoate, polyoxymethylene, low density polyethylene, high density polyethylene, polypropylene, ethylene-vinyl acetate, acrylonitrile styrene acrylate, epoxies, silicones, latex, natural organic materials or any other commodity bulk material, *e.g.*, commodity bulk plastic, or any combination or mixture thereof. For example, the base material may be nitrile rubber, which is a copolymer of acrylonitrile and butadiene. "Natural organic materials" may include, for example, agricultural products including materials made from crops and livestock, including but not limited to field crops, forage, cattle, sheep, hogs, goats, horses, poultry, and furbearing animals. Specific examples of materials from livestock include skins (*e.g.*, leather products) and furs.

The compound of Formula (I) may be provided such that it is present in the composition (i.e., that results from the combination with the base material and any other components) in an amount of about 0.5 to 15 wt%, based on the total weight of the composition. In some embodiments, the compound of Formula (I) is present in the composition in an amount of about 0.5-10, 1-10, 1-9, 1-8, 2-7, 3-7, 3-6, or 3-5 wt%. For example, the compound of Formula (I) may be present in an amount of about 0.5, 0.75, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, or 15 wt%.

Combining a compound according to Formula (I) with a base material in accordance with the present methods may be performed using any acceptable technique, such as mixing or blending. Those of ordinary skill in the art are able to identify suitable laboratory and industrial equipment for combining the compound with the base material, such as turbines, mixers (*e.g.,* cross-clearance, static, vacuum, jet, high viscosity, horizontal, intermix, turbo, planetary, banbury), blenders (*e.g.*, v-blenders, twin-screw, cone screw, double cone), or other suitable equipment.

Examples

The following examples are set forth so as to provide those of ordinary skill in the art with a complete disclosure and description of how the formulations, methods, and articles claimed herein may be developed and evaluated, and are intended to be purely exemplary of the invention and are not intended to limit the scope of what the inventors regard as their invention. nEfforts have been made to ensure accuracy with respect to numbers (e.g., amounts), but some errors and deviations should be accounted for.

***Example 1 - Preparation of Compound of Formula (I)***

A compound of Formula (I)

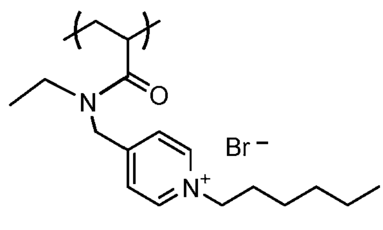

(I)

was prepared as follows in accordance with Scheme 1:

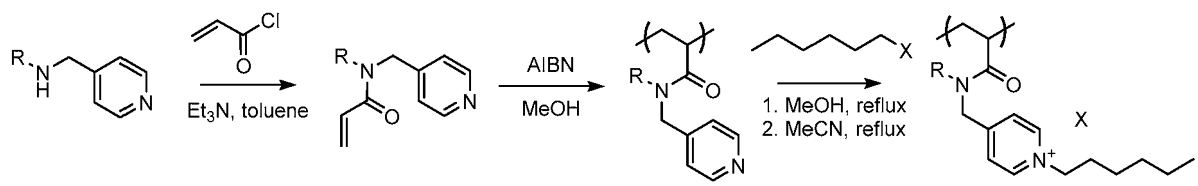


wherein R is $-CH_2CH_3$ and X is bromine.

First, the secondary amine precursor (6.81 g, 50.0 mmol) and triethylamine (7.3 mL, 52.5 mmol) were dissolved in toluene (100 mL) in a 250 mL 3-necked flask equipped with stir bar, $N_2$ inlet, and thermowell. The mixture was cooled to -12°C in an ice-methanol bath. Acryloyl chloride (4.06 mL, 50.0 mmol) was added dropwise. The first drop produced yellow chunks, so the remainder of acryloyl chloride was diluted into toluene (20 mL) and added dropwise under maximum stirring rate. The addition rate was controlled, and the cooling bath manually stirred to keep the internal temperature below -4°C throughout addition. The mixture was allowed to warm to near ambient temperature (7 °C) and vacuum-filtered to remove

precipitated $Et_3N$•HCl. The filter cake was washed with toluene (2 x 20 mL) and the combined filtrates were loaded onto a silica gel column (48 g) prepared in toluene. The product was eluted with EtOAc. The first fractions of desired product eluted just ahead of the EtOAc solvent front and were contaminated with a higher $R_f$ impurity. Yield of mixed fractions 1.48 g. The remainder of product eluted in pure EtOAc and was pure by TLC and NMR (except for residual EtOAc, which was removed by further drying overnight under slow rotation). Yield of pure fractions 4.50 g (47%) total yield 5.98 g (63%). Monomer 501PAL25 (pure fractions, 4.47 g, 23.5 mmol) was dissolved in methanol (45 mL) in a 100 mL 3-necked flask with inlet to $N_2$ line-bubbler, thermowell, and stir bar. The third neck was sealed with a polyethylene stopper, and the solution was sparged with $N_2$ using a needle inserted through the stopper. After 20 min, the solution was heated to 60°C. AIBN (19.3 mg, 117 μmol, 0.5 mol%) was added against a current of $N_2$, and the flask was sealed with a fresh polyethylene stopper. Manual agitation was required to wash down AIBN from flask neck. After 21 hours of stirring under $N_2$ at 60 °C, a sample of 0.2 mL was withdrawn by syringe, blown dry under $N_2$, and analyzed by $^1H$ NMR in $CDCl_3$, which indicated approx. 25% polymerization. A second sample (1 mL) was added dropwise to rapidly stirring MTBE (10 mL). The resulting gummy yellow precipitate was dissolved in DCM, blown dry, redissolved in DCM, taken into a syringe (total volume 0.3 mL) and reprecipitated in rapidly stirring MTBE (3 mL). The resulting white powdery precipitate was collected by filtration (cotton-plugged Pasteur pipette), washed with MTBE, dried under $N_2$ flow, and analyzed by $^1H$ NMR in $CDCl_3$ which showed only broad peaks consistent with polymer. A second portion of AIBN (19 mg, 0.5 mol%) was added, and the mixture was stirred at 60 °C for a further 24 h. NMR indicated just over 50% conversion. A third 0.5 mol% portion of AIBN was added, and the mixture was stirred at 60 °C for a further 45 h. After cooling, the mixture was concentrated to a foamy glassy solid. The residue was taken up in DCM and diluted to 16 mL, and a small sample was dried for NMR analysis which indicated 70% conversion. The remaining orange-brown solution was added dropwise to rapidly stirring MTBE (160 mL). After stirring for 10 min, the precipitate was collected by filtration, dried under $N_2$ flow, redissolved in DCM (15 mL) and reprecipitated in MTBE (150 mL). The product was collected by filtration and dried overnight under vacuum (75 mtorr) at room temperature. NMR in $CDCl_3$ indicated 0.39 equiv. (15% by mass) residual MTBE content. Further drying for 4 h at 45 °C reduced MTBE content to 12% based on mass loss and NMR in $CD_3OD$. Final yield 3.22 g (63% correcting for solvent content).

**Partially quaternized polymer:** Neutral pyridine polymer (2.4 g, 11.1 mmol monomer units) was dissolved in methanol (9 mL) and bromohexane (3.1 mL, 22 mmol) was added. The mixture was refluxed under $N_2$ for 14 h, and a sample was blown dry under $N_2$, further dried under vacuum, and analyzed by $^1H$ NMR in $CD_3OD$, which indicated just over 70% quaternization. After a further 25 h at 60 °C followed by 4 days at rt, NMR indicated complete consumption of free pyridine groups but with two sets of signals consistent with pyridinium groups. Samples of reaction mixture (0.2 mL each) were added to vials containing THF, EtOAc, or iPrOAc (3 mL of each solvent). EtOAc gave the most filterable precipitate. The latter was collected by filtration, dried under $N_2$ flow, and analyzed by $^1H$ NMR in $CD_3OD$, which again indicated 2 sets of pyridinium signals. $Et_3N$ (10 μL) was added to the NMR sample and the spectrum run again. The pyridinium signals diverged into sets consistent with N-hexylpyridinium (85%) and free pyridine (15%), indicating that the quaternization had halted due to competing protonation. A second sample of the rection mixture was treated with $Bu_3N$ (0.1 mL), diluted with MeOH until monophasic, and precipitated into EtOAc. NMR of the resulting precipitate (501PAL32p2) indicated the same pyridinium-free pyridine ratio as above, and $Bu_3N$ was not retained in the polymer. The remainder of the reaction mixture was treated with $Bu_3N$ (2.6 mL, 1 equiv.) and precipitated into rapidly stirring EtOAc (170 mL). The resulting gummy orange precipitate was separated by decantation, triturated with EtOAc until powdery, collected by filtration, dissolved in EtOH (9 mL) and precipitated into EtOAc (180 mL). The powdery precipitate was collected by filtration under $N_2$ and dried under rotation with scroll pump vacuum at 45 °C. NMR indicated 88% N-hexylpyridinium and 12% free pyridine groups. Yield 2.79 g (75%).

**Fully quaternized polymer:** Partially quaternized polymer (2.50 g, 7.45 mmol monomer units based on weighted average MW) was dissolved in MeCN (7.5 mL). Bromohexane (1.04 mL, 7.45 mmol) was added, and the mixture was refluxed under $N_2$ for 24.5 h. A sample was blown dry under $N_2$ and analyzed by NMR in $CD_3OD$, which was consistent with complete quaternization. This was confirmed by adding $Et_3N$ (10 μL), which resulted in no change to the aromatic region. The remainder of the reaction mixture was diluted with EtOH (2.5 mL) passed through a 0.45 μm syringe filter and precipitated in rapidly stirring EtOAc (180 mL). After stirring for 15 min, the product was collected by filtration under $N_2$, washed twice with EtOAc, and dried under $N_2$ flow. The resulting powder was further dried 2.5 h at 50 °C with rotation under vacuum (65 mtorr final) to give a light orange powder. Yield 2.39 g (90%). NMR indicated complete removal of solvents under these drying conditions.

### *Example 2 - Antimicrobial Efficacy*

A compound according to Formula (I), N-hexylpyridium-N-ethylacrylamide polymer, which is the compound that was formed according to the procedures described in Example 1, was tested in order to determine minimal inhibitory concentration (MIC), and minimal bacteriocidal concentration (MBC) against two bacterial species that are commonly associated with contamination of medical equipment and food processing and storage materials, *Salmonella aureus* (ATCC 6538) and *Pseudomonas aeruginosa* (ATCC 9027).

The test compound (produced in powder form) was reconstituted in sterile DI water. 0.25gm was mixed into solution with 2.0mL of sterile DI water for a 12.5% starting concentration.

Test inoculum was prepared by initiating the test microorganisms from library freezer stock into a 10.0mL of Tryptic Soy Broth (TSB) plate and incubated at 36.0 ± 1°C for 18-24 hours. Following incubation, the cultures were centrifuged and re-suspended in placed into 10.0mL of sterile Phosphate Buffered Saline (PBS). 0.200mL of the cultures were transferred to 9.0mL of sterile PBS to achieve a 0.5 McFarland Standard concentration of ~1.5 x $10^8$ CFU/mL. The cultures were further diluted in PBS to achieve a target concentration of 2 x $10^5$ to 8 x $10^5$ CFU/mL for the test inoculum by transferring 0.500mL of the raw culture at the McFarland Standard in 10.0mL of PBS to create the test inoculum. The prepared test inoculums were used within 30 minutes of finishing inoculum preparation.

*Broth Micro Dilution MIC.* Serial 1:1 dilutions (1 part test compound: 1 part diluent) were performed in a 96 well microtiter plate. Mueller Hinton Broth (MHB) was used as the well diluent. 0.100mL of MHB was added to wells 2-10. 0.200mL of the test compound was added to well 1, then diluted by taking 0.100mL from well 1 and adding it to well 2, and mixing. Then 0.100mL was taken from well 2 and added to well 3 then thoroughly mixed. The transferring of 0.100mL from the prior well to the following well was continued through well 10. 0.100mL was discarded from well 10 after the contents were thoroughly mixed. The Broth Micro Dilution MIC was performed in single replicate.

The prepared inoculum was added in an amount of 0.010mL to each well for the appropriate microorganism. The test inoculum was used to inoculate all wells within 30 minutes of diluting the culture for use in testing.

*Controls.* A positive control well was made in well 12 by placing 0.100mL MHB in the well and adding 0.010mL of the appropriated test inoculum to the broth and thoroughly mixing the well. The test substance was not added to the positive control well. A negative control well was made in well 11 by placing 0.200mL of MHB in the well. The negative well was not inoculated and test substance was not added to the well.

*Turbidity Control Wells.* Due to the turbidity of the test substances a control well was created for each test substance. The control wells contained the test substance and MHB in the same manner as described above in the Broth Micro Dilution MIC section, inoculum was not added to the wells.

*Time Zero Concentration of the Wells.* The time zero concentration of the wells was determined by removing 0.010mL from the positive well and adding it to 10.0mL of sterile PBS. Appropriate dilutions were plated to Tryptic Soy Agar.

*Media controls.* A purity streak of each test microorganism was performed on TSA. A sterility of all medias used in testing was performed on TSA.

*Neutralization Verification.* A neutralization effectiveness and neutralization toxicity test were performed prior to the MBC for each microorganism. The verifications were performed in double replicate. The neutralization verifications were only performed on the highest concentration of test substance used in testing. Neutralization Effectiveness Verification was performed by using the test microorganisms at the McFarland Standard. The test microorganisms were serially diluted in sterile PBS to achieve a concentration of ~10-100 CFU/mL in the neutralizer suspension. 0.900mL of sterile Dey-Engley Neutralizing Broth (D/E Broth) was inoculated with 0.010mL of the diluted test microorganism. And 0.100mL of the test substance was added to the inoculated D/E Broth suspension. The suspensions were allowed to dwell for >10 minutes, then the full tube was plated to TSA. Neutralizer Toxicity Verification was performed by using the diluted test microorganisms used for the neutralization effectiveness testing. 0.900mL of sterile Dey-Engley Neutralizing Broth (D/E Broth) was inoculated with 0.010mL of the diluted test microorganism and 0.100mL of PBS was added to the inoculated D/E Broth suspensions. The suspensions were allowed to dwell for >10 minutes, then the full tube was plated to TSA.

*Determining the Minimum Inhibitory Concentration (MIC).* Following incubation, the microtiter wells were visually examined for turbidity. Turbidity and/or the presence of a large microbial aggregation in the microtiter well indicates growth at a dilution. Growth was compared to the positive control well and control wells The MIC was determined to be the lowest concentration at which growth (turbidity) was not visually observed.

*Determining the Minimum Bactericidal Concentration (MBC).* 0.050mL was removed from the well corresponding to the observed MIC and two consecutive wells of greater test substance concentration. The aliquot that was removed from the well of the observed MIC and the two consecutive wells of greater test substance concentration were added to 0.900mL D/E Broth and vortex mixed. The suspension was pour plated with TSA. The entire contents of the microtiter tube with the D/E suspension was plated The MBC was the lowest concentration of test substance that demonstrated a 3 $\log_{10}$ reduction in CFU/mL in comparison to the time zero concentration of the wells.

Study success criteria were as follows: 1. Positive control well(s) are positive for growth; 2. Negative control well(s) are negative for growth; 3. All media sterility controls are negative for growth; 4. Purity streak displays pure growth of the target microorganism(s); 5. Concentration in the positive control well is between 2x105-8x105 CFU/mL; 6. The concentration of microorganisms recovered from the neutralizer effectiveness test should be ≥70% of the concentration recovered from the neutralizer toxicity test.

Performance success criteria were as follows: 1. The MIC is determined to be the well containing the lowest concentration of test substance that completely inhibits growth of the test microorganism; 2. The MBC is determined to be the lowest concentration of test substance that demonstrates a 99.9% reduction in CFU/mL when compared to the time

zero enumeration.

Relevant calculations were performed as follows:

$$\text{Log Reduction} = \text{Log } 10\ (B/A),$$

where A = surviving microbial concentration of the well, and B = initial numbers control concentration

$$\text{Percent Reduction} = (A-B)/A,$$

, where A = initial numbers control concentration, and where B = surviving microbial concentration of the well.

Table 1, below, provides the results of the evaluation concerning *S. aureus* ATCC 6538 percent reduction and log reduction for plated wells.

**Table 1**

| Test Microoganism | Well Number Rep 1 | CFU/mL | Percent Reduction of MBC vs. Initial Numbers Control | $Log_{10}$ Reduction of MBC vs. Initial Numbers Control |
|---|---|---|---|---|
| S. aureus (ATCC 6583) | T0 | 8.70E+04 | N/A | N/A |
| | 3 | <1.00E+00* | >99.99% | >4.94 |
| | 4 | <1.00E+00* | >99.99% | >4.94 |
| | 5 | <1.00E+00* | >99.99% | >4.94 |
| | 6 | <1.00E+00* | >99.99% | >4.94 |
| | 7 | <1.00E+00* | >99.99% | >4.94 |
| * Value is below limit of detection of detection of <1.00E+00 | | | | |

Table 2, below, provides the results of the evaluation concerning *P.aeruginosa* ATCC 9027 percent reduction and log reduction for plated wells.

**Table 2**

| Test Microoganism | Well Number Rep 1 | CFU/mL | Percent Reduction of MBC vs. Initial Numbers Control | $Log_{10}$ Reduction of MBC vs. Initial Numbers Control |
|---|---|---|---|---|
| *P.aeruginosa* ATCC 9027 | T0 | 7.95E+04 | N/A | N/A |
| | 4 | <1.00E+00* | >99.99% | >4.94 |
| | 5 | <1.00E+00* | >99.99% | >4.94 |
| | 6 | <1.00E+00* | >99.99% | >4.94 |
| | 7 | <1.00E+00* | >99.99% | >4.94 |
| | 8 | <1.00E+00* | >99.99% | >4.94 |
| * Value is below limit of detection of detection of <1.00E+00 | | | | |

Table 3, below, provides the measured MIC/MBC results against *S. aureus* ATCC 6538.

**Table 3**

| Replicate | Concentration of Test Substance (%) | | | | | | | | | |
|---|---|---|---|---|---|---|---|---|---|---|
| | 12.5 | 6.25 | 3.13 | 1.563 | 0.781 | 0.391 | 0.195 | 0.098 | 0.049 | 0.024 |
| 1 | N.T. | N.T. | N.T. | N.T. | N.T. | N.T. | MIC / MIBC | T. | T. | T. |

N.T. = No Turbidity
T. = Turbidity

Table 4, below, provides the measured MIC/MBC results against P. *aeruginosa* ATCC 9027.

**Table 4**

| Replicate | Concentration of Test Substance (%) | | | | | | | | | |
|---|---|---|---|---|---|---|---|---|---|---|
| | 12.5 | 6.25 | 3.13 | 1.563 | 0.781 | 0.391 | 0.195 | 0.098 | 0.049 | 0.024 |
| 1 | N.T. | N.T. | N.T. | N.T. | N.T. | N.T. | N.T. | MIC | MBC | T. |

N.T. = No Turbidity
T. = Turbidity

Table 5, below, provides the neutralization verification results against P. *aeruginosa* ATCC 9027.

Table 5

| Test | | Neutralization Validation plate counts (CFU) | Average CFU | % Recovery Neutralization Effectiveness | Neutralization Verified |
|---|---|---|---|---|---|
| | | | | Compared to Toxicity | |
| **Neutralization Effectiveness** | 81 | 90 | 85 | 87.96% | Yes |
| **Neutralization Toxicity** | 102 | 9 | 98 | N/A | |

Table 6, below, provides the neutralization verification results against *S. aureus* ATCC 6538.

**Table 6**

| Test | Neutralization Validation plate counts (CFU) | | Average CFU | % Recovery Neutralization Effectiveness Compared to Toxicity | Neutralization Verified |
|---|---|---|---|---|---|
| **Neutralization Effectiveness** | 70 | 73 | 71 | 72.96 | Yes |
| **Neutralization Toxicity** | 100 | 96 | 98 | N/A | |

Table 7, below, provides the results of an assessment of the control plates.

**Table 7**

| Control | Result |
|---|---|
| S. aureus ATCC 6538 Purity | Pure Growth |
| P. aeruginosa ATCC 9027 Purity | Pure Growth |
| Negative Control Well | No Turbidity |
| Positive Control Well | Turbidity |
| Mueller Hinton Broth Sterility | No Growth |
| Phosphate Buffer Saline Culture Diluent Sterility | No Growth |
| Phosphate Buffer Saline Dilution Sterility | No Growth |
| Dey-Engley Neutralizing Broth (D/E Broth) | No Growth |
| Tryptic Soy Agar Sterility | No Growth |

Table 8, below, provides the incubation conditions for the various test plates.

**Table 8**

| Incubation Temperature Range | Test Materials | Incubation Duration |
|---|---|---|
| 36 ± 1 °C | Media Sterility Plates, Time Zero Plates, Microorganism Purity Plates, MIC Plate, | 21 hours, 7 minutes |
| | and Neutralization Verification Plates | |
| 36 ± 1 °C | MBC Plates and Media Sterility Plates | 23 hours, 15 minutes |

***Example 3 - Antimicrobial Efficacy When Used With Plastic Materials***

Antimicrobial efficacy was also demonstrated in solid non-porous plastics, including polypropylene, which can be used in spunbound, spunblown, non-woven fabrics, and in woven textiles.

The Japanese Industrial Standard Committee (JIS) is an international organization that develops and standardizes test methods for a variety of products and materials. The JIS method Z 2801 is a quantitative test designed to assess the performance of antimicrobial finishes on hard, non-porous surfaces. The method can be conducted using contact times ranging from ten minutes up to 24 hours. For a JIS Z 2801 test, non-antimicrobial control surfaces are used as the baseline for calculations of microbial reduction. The method is versatile and can be used to determine the antimicrobial activity of a diverse array of surfaces including plastics, metals, and ceramics.

The JIS Z 2801 test procedure can be summarized as follows: a test microorganism is prepared, usually by growth in a liquid culture medium. The suspension of test microorganism is standardized by dilution in a nutritive broth, which affords microorganisms the opportunity to proliferate during the test. Control and test surfaces are inoculated with microorganisms, and then the microbial inoculum is covered with a thin, sterile film. Covering the inoculum spreads it, prevents it from evaporating, and ensures close contact with the antimicrobial surface. Microbial concentrations are determined at "time zero" by elution followed by dilution and plating to agar. A control is run to verify that the neutralization/elution method effectively neutralizes the antimicrobial agent in the antimicrobial surface being tested. Inoculated, covered control and antimicrobial test surfaces are allowed to incubate undisturbed in a humid environment for 24 hours, usually at body temperature. After incubation, microbial concentrations are determined. Reduction of microorganisms relative to the control surface is calculated.

For a JIS Z 2801 study to be scientifically defensible, the following criteria must be met: (1) the average number of viable bacteria recovered from the time zero samples must be approximately 1 x $10^4$ cells/$cm^2$ or greater; (2) ordinary consistency between replicates must be observed for the time zero samples; (3) the number of viable bacteria recovered from the control surface after the contact time must not be significantly (>2-Log10) less than the original inoculum concentration; (4) positive / growth controls must demonstrate growth of appropriate test microorganism; (5) negative / purity controls must demonstrate no growth of test microorganism.

Passing criteria specifies a performance criteria for antimicrobial efficacy of greater than or equal to a 2 Log10 or 99% reduction in the test microorganisms when comparing the treated surface to the control surface after the contact time.

Table 9, below, shows the bactericidal efficacy of polypropylene compounded with 6% wt/wt N-hexylpyridium-N-ethylacrylamide polymer, used as an additive, against *S. aureus* and *P. aeruginosa* over 24 h. This material has demonstrated in excess of three log reduction against the test organisms, with an inoculation time of 24 h, while still being processable into non-woven fabrics.

**Table 9**

| Test Microorganism | Contact Time | Carrier | PFU/Carrier | Percent Reduction Compared to 24h Control | Log10 Reduction Compared to 24h Control |
|---|---|---|---|---|---|
| *S. aureus* ATCC 6538 | Time Zero | Control | 2.45E+05 | N/A | |
| | 24 hours | Control | 2.40E+04 | N/A | |
| | 24 hours | Polypropylene 6% loaded | <5.00+00* | >99.98 | >3.68 |
| *P. aeruginosa* ATCC 9027 | Time Zero | Control | 1.10E+05 | N/A | |
| | 24 hours | Control | 2.00E+05 | N/A | |
| | 24 hours | Polypropylene 6% loaded | 3.50E+01 | 99.998 | 4.76 |
| *Limit of detection. Plate counts fell below the limit of detection of <5.00E+00 CFU/Carrier for *S. aureus* | | | | | |

***Example 4 - Antimicrobial Efficacy When Used With Porous Materials***

As demonstrated below, the tested compound according to Formula (I), N-hexylpyridium-N-ethylacrylamide polymer, can also be used to impose antimicrobial properties into porous materials, such as rawhide leather.

Treated leather samples were prepared by soaking in a mixture of 3% N-hexylpyridium-N-ethylacrylamide polymer in ethanol for 10 minutes and then allowed to dry for 24 h. Table 10, below, shows the results of the testing, specifically the bactericidal efficacy of a panel of pathogens at 24 h inoculation time. In many cases the log reduction exceeded the limits of the test.

**Table 10**

| Test Microorganism | Contact Time | Carrier | PFU/Carrier | Percent Reduction Compared to 24h Control | Log10 Reduction Compared to 24h Control |
|---|---|---|---|---|---|
| *K. pneumoniae* ATCC 4352 | Time Zero | Control | 2.60E+05 | N/A | |
| | 24 hours | Control | 2.30E+05 | N/A | |
| | | Treated Leather | <5.00+00* | >99.998 | >4.66 |
| *P. aeruginosa* ATCC 9027 | Time Zero | Control | 2.25E+05 | N/A | |
| | 24 hours | Control | 1.80E+06 | N/A | |
| | | Treated Leather | <5.00+00* | >99.9997 | >5.56 |
| *S. enterica* ATCC 10708 | Time Zero | Control | 2.40E+05 | N/A | |
| | 24 hours | Control | 1.75E+06 | N/A | |
| | | Treated Leather | <5.00+00* | >99.997 | >5.54 |
| *E. coli* ATCC 8739 | Time Zero | Control | 1.10E+05 | N/A | |
| | 24 hours | Control | 3.05E+06 | N/A | |
| | | Treated Leather | <5.00+00* | >99.9998 | >5.97 |

(continued)

| Test Microorganism | Contact Time | Carrier | PFU/Carrier | Percent Reduction Compared to 24h Control | Log10 Reduction Compared to 24h Control |
|---|---|---|---|---|---|
| *B. cereus* ATCC 14579 | Time Zero | Control | 7.50E+04 | N/A | |
| | 24 hours | Control | 4.40E+05 | N/A | |
| | | Treated Leather | <5.00+00* | >99.9989 | >4.94 |
| *S. pneumoniae* ATCC 49619 | Time Zero | Control | 7.50E+04 | N/A | |
| | 24 hours | Control | 4.40E+05 | N/A | |
| | | Treated Leather | <5.00+00* | >99.9989 | >4.94 |
| *S. aureus* ATCC 33592 (MRSA) | Time Zero | Control | 3.30E+05 | N/A | |
| | 24 hours | Control | 2.80E+05 | N/A | |
| | | Treated Leather | <5.00+00* | >99.998 | >4.75 |
| *C. albicans* ATCC 10231 | Time Zero | Control | 1.05E+05 | N/A | |
| | 24 hours | Control | 1.55E+05 | N/A | |
| | | Treated Leather | 4.30E+02 | 99.72 | 2.56 |
| *A. brasiliensis* ATCC 9642 | Time Zero | Control | 1.45E+05 | N/A | |
| | 24 hours | Control | 2.45E+05 | N/A | |
| | | Treated Leather | 6.20E+02 | 99.75 | 2.60 |
| *C. auris* CDC AR Bank 381 | Time Zero | Control | 4.75E+05 | N/A | |
| | 24 hours | Control | 4.45E+05 | N/A | |
| | | Treated Leather | 1.05E+05 | 76.4 | 0.63 |
| *indicates plate counts fell below the detectable limit of the test | | | | | |

## Claims

**1.** A compound of Formula (I):

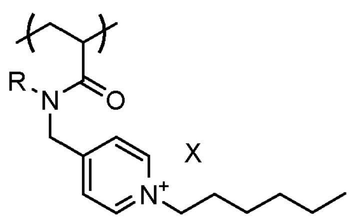

(I)

wherein

R is H, -OH, $-CH_2CH_3$, $-(CH_2)_2CH_3$, $-(CH_2)_3CH_3$, or $-(CH_2)_2OCH_3$; and,
X is a negative counterion that is bromide, chloride, fluoride, or a phosphate,
wherein the compound is a homopolymer.

**2.** The compound according to claim 1, wherein X is bromide, and/or wherein R is $-CH_2CH_3$.

**3.** A mixture comprising at least two different compounds according to claim 1.

**4.** A composition comprising a compound according to claim 1 and a base material.

**5.** The composition according to claim 4, wherein X is bromide, and/or wherein R is $-CH_2CH_3$.

**6.** The composition according to any one of claims 4-5, wherein the base material is polyvinyl chloride, chlorinated polyvinyl chloride, polycarbonate, nylon 6-6, polyethylene terephthalate, polyethylene terephthalate glycol, polybutylene terephthalate, polyether block amide, acrylate, acrylonitrile butadiene styrene, polystyrene, polylactic acid, polyhydroxyalkanoate, polyoxymethylene, low density polyethylene, high density polyethylene, polypropylene, ethylene-vinyl acetate, acrylonitrile styrene acrylate, an epoxy, a silicone, latex, a natural organic material, or any combination thereof, optionally wherein the base material is polyvinyl chloride.

**7.** The composition according to any one of claims 4-6, wherein the compound of Formula (I) is present in the composition in an amount of about 1-10 wt%, or wherein the compound of Formula (I) is present in the composition in an amount of about 2-7 wt%, or wherein the compound of Formula (I) is present in the composition in an amount of about 3-5 wt%.

**8.** An article comprising a composition according to any one of claims 4-6.

**9.** The article according to claim 8 comprising a disposable glove, a mask, a medical gown, a shoe cover, a medical cap, medical tubing, a delivery device, a storage container, an implantable device, an implantable medical lead, a textile, or a sponge, or wherein the article comprises a paint, coating, sealant, or wax.

**10.** A method for preparing a compound of Formula (I)

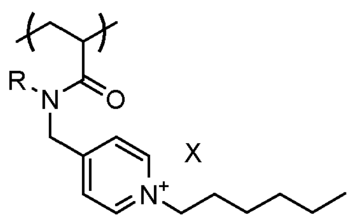

(I)

wherein

R is H, -OH, $-CH_2CH_3$, $-(CH_2)_2CH_3$, $-(CH_2)_3CH_3$, or $-(CH_2)_2OCH_3$; and,
X is a negative counterion that is bromide, chloride, fluoride, or a phosphate,

wherein said method is according to Scheme 1:

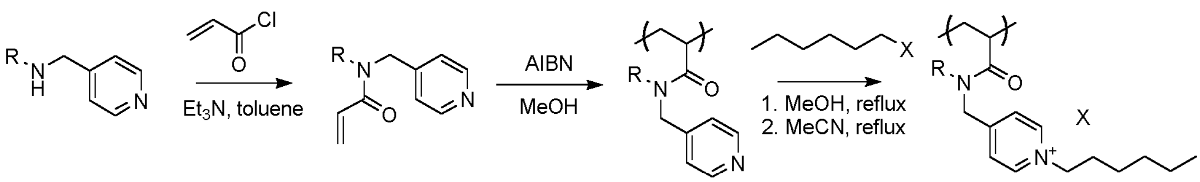


**Scheme 1**.

**11.** The method according to claim 10, wherein X is bromide, and/or wherein R is $-CH_2CH_3$.

**12.** A method comprising combining a base material with a compound of a Formula (I)

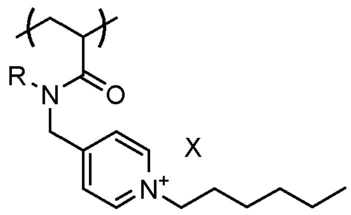

(I)

wherein

R is H, -OH, $-CH_2CH_3$, $-(CH_2)_2CH_3$, $-(CH_2)_3CH_3$, or $-(CH_2)_2OCH_3$; and,
X is a negative counterion that is bromide, chloride, fluoride, or a phosphate.

13. The method according to claim 12, wherein X is bromide, and/or wherein R is $-CH_2CH_3$, and/or wherein the base material is polyvinyl chloride, chlorinated polyvinyl chloride, polycarbonate, nylon 6-6, polyethylene terephthalate, polyethylene terephthalate glycol, polybutylene terephthalate, polyether block amide, acrylate, acrylonitrile butadiene styrene, polystyrene, polylactic acid, polyhydroxyalkanoate, polyoxymethylene, low density polyethylene, high density polyethylene, polypropylene, ethylene-vinyl acetate, acrylonitrile styrene acrylate, an epoxy, a silicone, latex, or any combination thereof.

## Patentansprüche

1. Verbindung der Formel (I):

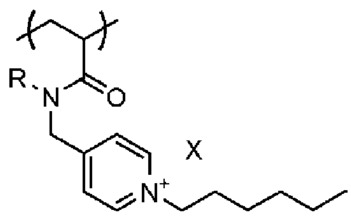

(I)

wobei

R H, -OH, $-CH_2CH_3$, $-(CH_2)_2CH_3$, $-(CH_2)_3CH_3$, oder $-(CH_2)_2OCH_3$ ist; und
X ein negatives Gegenion ist, das Bromid, Chlorid, Fluorid oder ein Phosphat ist,
wobei die Verbindung ein Homopolymer ist.

2. Verbindung nach Anspruch 1, wobei X Bromid ist und/oder wobei R $-CH_2CH_3$ ist.

3. Gemisch umfassend wenigstens zwei verschiedene Verbindungen nach Anspruch 1.

4. Zusammensetzung umfassend eine Verbindung nach Anspruch 1 und ein Grundmaterial.

5. Zusammensetzung nach Anspruch 4, wobei X Bromid ist und/oder wobei R $-CH_2CH_3$ ist.

6. Zusammensetzung nach einem der Ansprüche 4-5, wobei das Grundmaterial Polyvinylchlorid, chloriertes Polyvinylchlorid, Polycarbonat, Nylon 6-6, Polyethylenterephthalat, Polyethylenterephthalatglycol, Polybutylenterephthalat, Polyetherblockamid, Acrylat, Acrylnitril-Butadien-Styrol, Polystyrol, Polymilchsäure, Polyhydroxyalkanoat, Polyoxymethylen, Polyethylen mit niedriger Dichte, Polyethylen mit hoher Dichte, Polypropylen, Ethylenvinylacetat, Acrylnitrilstyrolacrylat, ein Epoxid, ein Silicon, Latex, ein natürliches organisches Material oder eine beliebige Kombination davon ist, wobei das Grundmaterial gegebenenfalls Polyvinylchlorid ist.

7. Zusammensetzung nach einem der Ansprüche 4-6, wobei die Verbindung der Formel (I) in der Zusammensetzung in einer Menge von etwa 1-10 Gew.-% vorhanden ist oder wobei die Verbindung der Formel (I) in der Zusammensetzung in einer Menge von etwa 2-7 Gew.-% vorhanden ist oder wobei die Verbindung der Formel (I) in der Zusammensetzung in einer Menge von etwa 3-5 Gew.-% vorhanden ist.

**8.** Gegenstand umfassend eine Zusammensetzung nach einem der Ansprüche 4-6.

**9.** Gegenstand nach Anspruch 8, umfassend einen Einweghandschuh, eine Maske, ein medizinisches Kleidungsstück, einen Überschuh, eine medizinische Haube, medizinische Schläuche, eine Abgabevorrichtung, einen Aufbewahrungsbehälter, eine implantierbare Vorrichtung, eine implantierbare medizinische Leitung, ein Textilstück oder einen Schwamm, oder wobei der Gegenstand einen Anstrich, eine Beschichtung, ein Dichtungsmittel oder ein Wachs umfasst.

**10.** Verfahren zur Herstellung einer Verbindung der Formel (I)

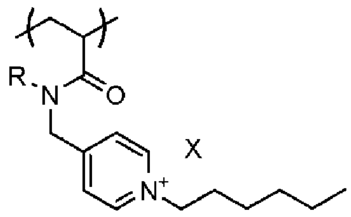

(I)

wobei

R H, -OH, $-CH_2CH_3$, $-(CH_2)_2CH_3$, $-(CH_2)_3CH_3$, oder $-(CH_2)_2OCH_3$ ist; und
X ein negatives Gegenion ist, das Bromid, Chlorid, Fluorid oder ein Phosphat ist,
wobei das Verfahren gemäß Schema 1 ist:

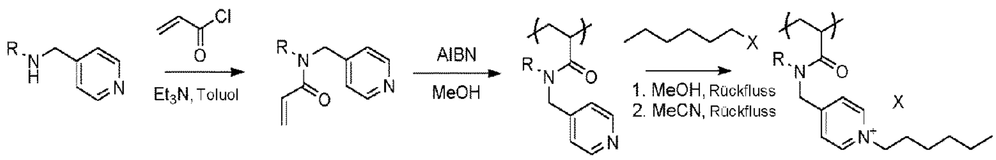


Schema 1.

**11.** Verfahren nach Anspruch 10, wobei X Bromid ist und/oder wobei R $-CH_2CH_3$ ist.

**12.** Verfahren umfassend Kombinieren eines Grundmaterials mit einer Verbindung der Formel (I)

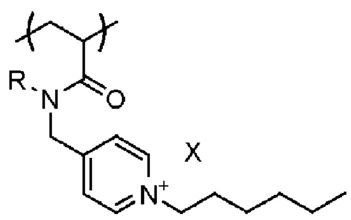

(I)

wobei

R H, -OH, $-CH_2CH_3$, $-(CH_2)_2CH_3$, $-(CH_2)_3CH_3$, oder $-(CH_2)_2OCH_3$ ist; und
X ein negatives Gegenion ist, das Bromid, Chlorid, Fluorid oder ein Phosphat ist.

**13.** Verfahren nach Anspruch 12, wobei X Bromid ist und/oder wobei R $-CH_2CH_3$ ist und/oder wobei das Grundmaterial Polyvinylchlorid, chloriertes Polyvinylchlorid, Polycarbonat, Nylon 6-6, Polyethylenterephthalat, Polyethylenterephthalatglycol, Polybutylenterephthalat, Polyetherblockamid, Acrylat, Acrylnitril-Butadien-Styrol, Polystyrol, Polymilchsäure, Polyhydroxyalkanoat, Polyoxymethylen, Polyethylen mit niedriger Dichte, Polyethylen mit hoher Dichte, Polypropylen, Ethylenvinylacetat, Acrylnitrilstyrolacrylat, ein Epoxid, ein Silicon, Latex oder eine beliebige Kombination davon ist.

## Revendications

1. Composé de formule (I) :

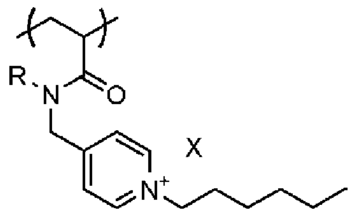

(I)

dans laquelle

R est H, -OH, $-CH_2CH_3$, $-(CH_2)_2CH_3$, $-(CH_2)_3CH_3$, ou - $(CH_2)_2OCH_3$ ; et,
X est un contre-ion négatif qui est bromure, chlorure, fluorure ou un phosphate,
dans lequel le composé est un homopolymère.

2. Composé selon la revendication 1, dans lequel X est bromure et/ou dans lequel R est $-CH_2CH_3$.

3. Mélange comprenant au moins deux composés différents selon la revendication 1.

4. Composition comprenant un composé selon la revendication 1 et une matière de base.

5. Composition selon la revendication 4, dans laquelle X est bromure et/ou dans laquelle R est $-CH_2CH_3$.

6. Composition selon l'une quelconque des revendications 4 à 5, dans laquelle la matière de base est un poly(chlorure de vinyle), un poly(chlorure de vinyle) chloré, un polycarbonate, un nylon 6-6, un poly(téréphtalate d'éthylène), un poly(téréphtalate d'éthylène) glycol, un poly(téréphtalate de butylène), un polyéther bloc amide, un acrylate, un acrylonitrile butadiène styrène, un polystyrène, un poly(acide lactique), un polyhydroxyalcanoate, un polyoxyméthylène, un polyéthylène basse densité, un polyéthylène haute densité, un polypropylène, un éthylène-acétate de vinyle, un acrylonitrile styrène acrylate, un époxy, une silicone, un latex, une matière organique naturelle, ou une quelconque combinaison de ceux-ci, éventuellement, dans laquelle la matière de base est un poly(chlorure de vinyle) .

7. Composition selon l'une quelconque des revendications 4 à 6, dans laquelle le composé de formule (I) est présent dans la composition en une quantité d'environ 1 à 10 % en poids, ou dans laquelle le composé de formule (I) est présent dans la composition en une quantité d'environ 2 à 7 % en poids, ou dans laquelle le composé de formule (I) est présent dans la composition en une quantité d'environ 3 à 5 % en poids.

8. Article comprenant une composition selon l'une quelconque des revendications 4 à 6.

9. Article selon la revendication 8, comprenant un gant jetable, un masque, une blouse médicale, un couvre-chaussure, un capuchon médical, un tube médical, un dispositif d'administration, un récipient de stockage, un dispositif implantable, un fil médical implantable, un textile ou une éponge, ou dans lequel l'article comprend une peinture, un revêtement, un agent d'étanchéité ou une cire.

10. Procédé pour la préparation d'un composé de formule (I)

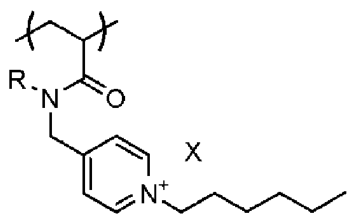

(I)

dans laquelle

R est H, -OH, $-CH_2CH_3$, $-(CH_2)_2CH_3$, $-(CH_2)_3CH_3$, ou - $(CH_2)_2OCH_3$ ; et,
X est un contre-ion négatif qui est bromure, chlorure, fluorure ou un phosphate,
dans lequel ledit procédé est selon le schéma 1 :

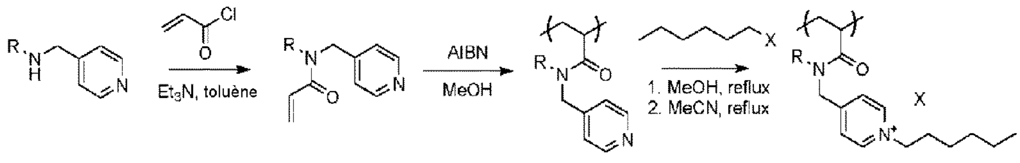


schéma 1.

**11.** Procédé selon la revendication 10, dans lequel X est bromure et/ou dans lequel R est $-CH_2CH_3$.

**12.** Procédé comprenant la combinaison d'une matière de base avec un composé d'une formule (I)

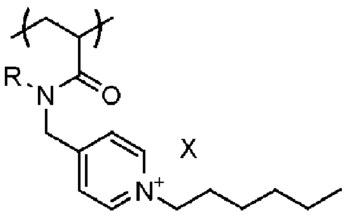
(I)

dans laquelle

R est H, -OH, $-CH_2CH_3$, $-(CH_2)_2CH_3$, $-(CH_2)_3CH_3$, ou - $(CH_2)_2OCH_3$ ; et,
X est un contre-ion négatif qui est bromure, chlorure, fluorure ou un phosphate.

**13.** Procédé selon la revendication 12, dans lequel X est bromure, et/ou dans lequel R est $-CH_2CH_3$, et/ou dans lequel la matière de base est un poly(chlorure de vinyle), un poly(chlorure de vinyle) chloré, un polycarbonate, un nylon 6-6, un poly(téréphtalate d'éthylène), un poly(téréphtalate d'éthylène) glycol, un poly(téréphtalate de butylène), un polyéther bloc amide, un acrylate, un acrylonitrile butadiène styrène, un polystyrène, un poly(acide lactique), un polyhydroxyalcanoate, un polyoxyméthylène, un polyéthylène basse densité, un polyéthylène haute densité, un polypropylène, un éthylène-acétate de vinyle, un acrylonitrile styrène acrylate, un époxy, une silicone, un latex, ou une quelconque combinaison de ceux-ci.